(19) Europäisches Patentamt
European Patent Office
Office européen des brevets

(11) Publication number: **0 362 730 A1**

(12) # EUROPEAN PATENT APPLICATION

(21) Application number: 89118142.2

(22) Date of filing: 30.09.89

(51) Int. Cl.5: C07D 493/20 , A61K 31/35 , //(C07D493/20,323:00,321:00, 311:00)

(30) Priority: 04.10.88 EP 88116378

(43) Date of publication of application: 11.04.90 Bulletin 90/15

(84) Designated Contracting States: AT BE CH DE ES FR GB IT LI NL

(71) Applicant: HOECHST AKTIENGESELLSCHAFT
Postfach 80 03 20
D-6230 Frankfurt am Main 80(DE)

(72) Inventor: Venugopalan, Bindumadhavan, Dr.
Building No. 43/A Flat No. 31 Brindavan Society
P.O. Thane Maharashtra(IN)
Inventor: Bapat, Chintamam Prabhakar, Dr.
Hoechst Quarters Darga Road
Mulund (West) Bombay 400 082(IN)
Inventor: Karnik, Pravin Jayant, Dr.
Bld. 22/A Flat No. 23 Brindavan Society
Thane 400 061(IN)
Inventor: Lal, Bansi, Dr.
30A. Advani Apartments
Mulund (West) Bombay 400 080(IN)
Inventor: Chatterjee, Deepak Kumar, Dr.
Sheetal Bungalow No. 2 Mahatma Phule Road
Mulund (East) Bombay 400 081(IN)
Inventor: Iyer, Subramani Natrajan, Dr.
A/4 Amrachhaya Ashok Nagar Nabur
Mulund (West) Bombay 400 080(IN)
Inventor: Rupp, Richard Helmut, Dr.
Roederweg 16a
D-6240 Königstein/Taunus(DE)

(54) **Novel artemisinin derivatives, processes for their preparation and their use as antiprotozoal agents.**

(57) 10-Substibuted ethers and thioether derivatives of dihydroquinghaosu of the formula I

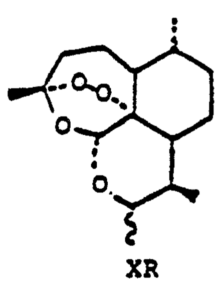

I

wherein X and R have the given meaning, are active against protozoal infections, especially against Malaria.

EP 0 362 730 A1

## Novel Artemisinin derivatives, processes for their preparation and their use as antiprotozoal agents

This invention relates to 10-substituted ether and thioether derivatives of 3α,12α-epoxy-3,4,5,5aα,6,7,8aα,9,10,12β,12a-dodecahydro-10-hydroxy-3β,6α,9β-trimethylpyrano(4,3-j)(1,2)benzodioxepin, also known as Dihydroartemisinin or Dihydroquinghaosu (DHQ) and pharmaceutically acceptable salts thereof, processes for their preparation and their use as chemotherapeutics against protozoal infections.

Artemisinin and its ethers are reviewed in the following publications:

Medicinal Research Reviews, Vol. 7, No. 1, 29-52 (1987) J. of Medicinal Chemistry, 31, 645 (1988).

Although compounds of the prior art have been reported to possess antimalarial activity, they have invariably had to be administered parenterally for activity to be demonstrated at sufficiently low doses. Recrudescence was also observed at a rate of 10 - 30 % in a month after administration with such parenteral forms. The use of compounds is thus claimed only for cerebral malaria. Surprisingly it has now been determined that the novel derivatives of artemisinin described herein are characterized by two special qualities:

(a) they possess potent antimalarial activity when administered orally to animals becoming thus potential agents for all forms of malaria resulting from susceptible and resistant forms of pathogenic Plasmodium strains and

(b) they possess antiprotozoal activity in general and in particular antiamoebic activity against Entamoeba histolytica and anticoccidial activity against the protozoa Eimera tenella, hitherto not known for artemisinin or any of its known derivatives.

Thus the instant invention is directed to 10-substituted ethers and thioether derivatives of dihydroquinghaosu as represented by the general formula I,

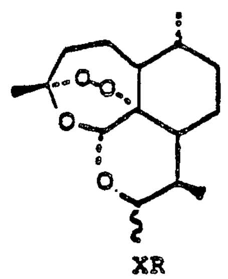

I

wherein X stands for oxygen, sulphur, SO or $SO_2$ when X stands for sulphur, SO or $SO_2$; R stands for $C_1$-$C_8$ alkyl, $C_4$-$C_8$ cycloalkyl, substituted alkyl, alkenyl, substituted alkenyl, alkynyl, substitutéd alkynyl; aryl, aralkyl, alkylsulfinyl, heterocyclic alkyl, a group

$$-(\underset{R^1}{\overset{|}{CH}})_{m_1}-(CH_2)_{n_1}-(\underset{R^2}{\overset{|}{CH}})_{m_2}-(CH_2)_{n_2}-(\underset{R^3}{\overset{|}{CH}})_{m_3}-(CH_2)_{n_3}-Y$$

wherein two neighbouring carbon atoms can be connected by a double or a triple bond and
wherein $R^1$ stands for hydrogen, alkyl;
$R^2$ and $R^3$ stand for hydrogen, hydroxy, alkyl,
Y stands for nitrile, aryl or a group

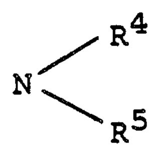

wherein $R^4$, $R^5$ when they are same stand for hydrogen, alkyl, substituted alkyl, when $R^4$ stands for hydrogen, $R^5$ stands for alkyl, substituted alkyl, aryl, aralkyl; when $R^4$ and $R^5$ together with the nitrogen atom to which they are attached form a heterocycle, this heterocycle may contain an additional heteroatom and may optionally be substituted at one or more places,
$m_1$-$m_3$ stand for 0 or 1 and $n_1$-$n_3$ stand for integer 0 - 9 with the proviso that $m_1$-$m_3$ and $n_1$-$n_3$ do not stand

simultaneously for 0.

When X stands for oxygen, R stands for 3-hydroxypropyl, acetoxy ethyl, oxypropyl, 2,3-oxypropyl, bis isopropoxypropyl, ethylnitrile, 3-methyl-1-pentynyl, heterocyclic alkyl, 2-hydroxyethyloxyethyl or a group

$$-(\underset{R^1}{\overset{|}{CH}})_{m_1}-(CH_2)_{n_1}-(\underset{R^2}{\overset{|}{CH}})_{m_2}-(CH_2)_{n_2}-(\underset{R^3}{\overset{|}{CH}})_{m_3}-(CH_2)_{n_3}-Y$$

which has the meaning as defined above or an aryl group and pharmaceutically acceptable salts thereof with the exception of those compounds in which X is O and R is benzyl, 4-carboxybenzyl, 3-fluorobenzyl, phenyl and phenyl substituted by methyl, methyloxy, ethyloxy, halogen, trichloromethyl or tribromomethyl.

In the formulae presented herein the various substituents are illustrated as joined to pyrano(4,3-j)(1,2)-benzodioxepin nucleus by one of two notations, a solid line (-) indicating a substituent which is in the β-orientation (i.e. above the plane of molecule) and a broken line (---) indicating a substituent which is in the α-orientation (i.e. below the plane of the molecule). The formulae have all been drawn to show the compounds in their absolute configuration. In as much as the starting materials having pyrane (4,3-j)(1,2)-benzodioxepin nucleus are naturally occurring or are derived from naturally occurring materials, they as well as the final products have a pyrano(4,3-j)(1,2)benzodioxepin nucleus in the single absolute configuration depicted herein. The processes of the present invention, however, are intended to apply as well to the synthesis of pyrano(4,3-j)(1,2)benzodioxepines of the racemic series.

In addition to the optical centers of pyrano(4,3-j)(1,2)benzodioxepin nucleus, the substituents thereon may also contain chiral centers contributing to the optical properties of the compounds of the present invention and providing a means for the resolution thereof by conventional methods, for example, by the use of optically active acids. A wavy line (~) indicates that substituents can either be in the α-orientation or β-orientation. The present invention comprehends all optical isomers and racemic forms of the compounds of the present invention where such compounds have chiral centers in addition to those of the pyrano(4,3-j)-(1,2)benzodioxepin nucleus.

The term alkyl stands for $C_1$-$C_8$ straight or branched chain carbon compounds such as methyl, ethyl, propyl, butyl, isopropyl, t-butyl. The term alkenyl stands for straight or branched chain carbon compounds containing one or more double bonds. Suitable examples are acrylyl, stearyl, cinnamyl.

The term alkynyl stands for straight or branched chain carbon compounds containing one or more triple bonds and may in addition contain a double bond. Examples of alkynyl groups are 3-methyl-1-pentoynyl, 1-butynyl, 3-methyl-1-butynyl, 2-butynyl-1-hydroxymethyl.

Substituents of substituted alkyl, alkenyl and alkynyl are halogen, hydroxy, carboxy, nitrile, acyl, aryl, heterocycle or a group $NR^4R^5$ wherein $R^4$ and $R^5$ are as defined above.

The term aryl stands for a phenyl group which is optionally substituted by one or more substituents such as substituted alkyl, alkenyl and alkynyl, halogen, nitro, amino, hydroxy, alkoxy, carboxy, alkylcarboxylate, trifluoromethyl, substituted amino, acetyl, alkenyloxy, alkynyloxy. When X stands for S the term halogen substituent on aryl group stands for fluoro, chloro, bromo, iodo but when X stands for oxygen, halogen substituents stands for chloro, bromo, and iodo. The term heterocycle or heterocyclic stands for cyclic compounds containing one or more heteroatoms such as piperazino, morpholino, piperidino, pyrrolidino, phthalimido, isoxazolyl, furanyl, tetrahydrofuranyl, optionally substituted at one or more places by alkyl, alkoxy, hydroxy, halogen and/or aryl groups.

Preferred compounds of the invention are listed in Table 1 and Table 2.

**Table 1**

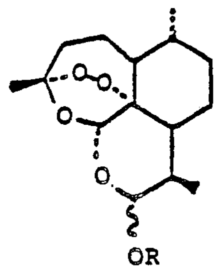

| R | | m.p. (°C) | Solvent for crystallisation | salt | Yield (%) |
|---|---|---|---|---|---|
| $CH_2CN$ | (β) | 150-152 | methylenechlorid/ pet.ether | - | 11 |
| 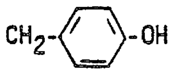 | (β) | oil | - | - | 41 |
| 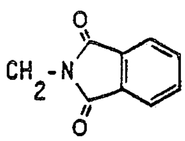 | (α+β) | oil | - | - | 27 |
| $CH_2COCH_3$ | (β) | 106-107 | pet.ether | - | 24 |
| $CH_2CH_2CN$ | (β) | 137-138 | methylene chloride/ pet.ether | - | 67 |
| $CH_2CH_2OCH_2CH_2OH$ | (α) | oil | - | - | 19 |

**Table 1** (Contd.)

| R | | m.p. (°C) | Solvent for crystallisation | salt | Yield (%) |
|---|---|---|---|---|---|
| $CH_2CH_2OCH_2CH_2OH$ | (β) | oil | - | - | 38 |
| $CH_2CH_2NH_2$ | (β) | 141-143 (d) | diisopropyl ether | maleate | 14 |
| 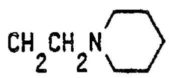 | (β) | 62- 64 | n-pentane | - | 59 |
| 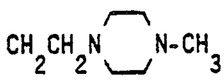 | (β) | 71- 73 | pet.ether | hydrochloride | 71 |
| 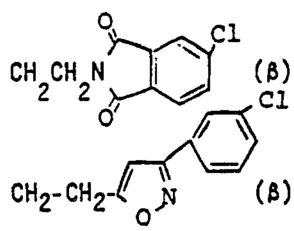 | (β) | 150-152 | chloroform/ diisopropyl ether | - | 32 |
| | (β) | 146-148 | methylchloride pet.ether | - | 22 |
| 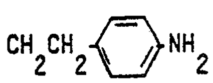 | (β) | 152-154 | diisopropyl ether | hydrochloride | 26 |
| 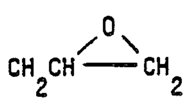 | (β) | 55- 57 | n-pentane | - | 25 |
| $CH_2CH_2CH_2OH$ | (β) | 74- 74 | pet.ether | - | 21 |
| $CH_2CH_2CH_2NH_2$ | (β) | 138-140 | methanol/ diisopropyl ether | maleate | 22 |

Table 1 (Contd.)

| R | | m.p. (°C) | Solvent for crystallisation | salt | Yield (%) |
|---|---|---|---|---|---|
| $CH_2-CH(OH)-CH_3$ | (α) | oil | - | - | 11 |
| $CH_2CH(OH)-CH_3$ | (β) | oil | - | - | 30 |
| $CH(CH_3)CH_2CH_2NH_2$ | (α+β) | 92- 94 | diisopropyl ether | maleate | 20 |
| $CH_2CH(OH)CH_2N(C_2H_5)_2$ | (β) | oil | - | hydrochloride | 70 |
| 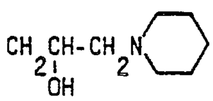 | (β) | oil | - | hydrochloride | 40 |
| 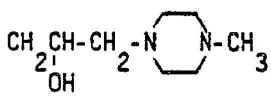 | (β) | oil | - | hydrochloride | 41 |
| $CH_2CH(NH_2)CH_2CH_3$ | | oil | - | maleate | 18 |
| 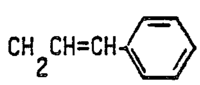 | (β) | oil | - | - | 57 |
| 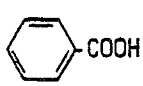 | (α+β) | 167-171 | ether/pet.ether | - | 76 |

**Table 1 (Contd.)**

| R | m.p. (°C) | Solvent for crystallisation | salt | Yield (%) |
|---|---|---|---|---|
| 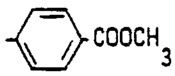 (α+β) | 145-147 | methylene chloride/ pet.ether | - | 47 |
| 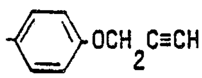 (β) | oil | - | - | 48 |
| 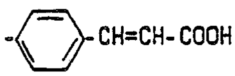 (β) | 146-148 | methylene chloride/ pet.ether | - | 79 |
| 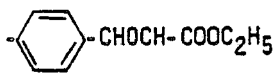 (β) | 137-139 | pet.ether (40-60) | - | 19 |
| 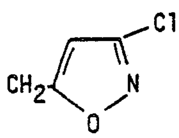 (β) | 105 | - | - | 53 |
| 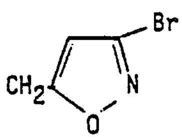 (β) | oil | - | - | 63 |
| 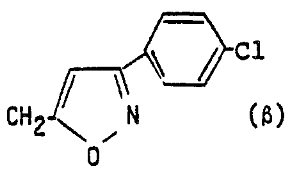 (β) | oil | - | - | 47 |
| 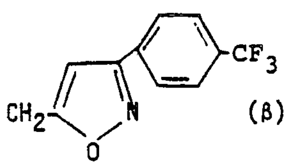 (β) | 117-118 | methylene chloride/ pet.ether | - | 37 |

Table 1 (Contd.)

| R | m.p. (°C) | Solvent for crystallisation | salt | Yield (%) |
|---|---|---|---|---|
| 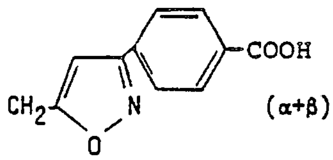 | (α+β)150-152 | methylene chloride/ pet.ether | - | 99 |
| 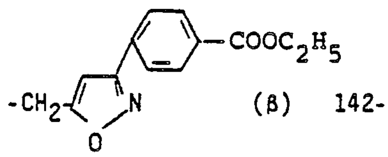 | (β) 142-144 | methylene chloride/ pet.ether | - | 55 |
| $CH_2C{\equiv}CCH_2OH$ | oil | - | - | 40 |
| $CH(CH_3)C{\equiv}CH$ | (β) 91-93 | pet.ether | - | 35 |
| $C(CH_3)_2C{\equiv}CH$ | (β) 58-60 | pet.ether | - | 57 |
| $CH_2NHCOCH_2$- 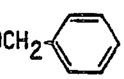 | (α+β) 163-164 | pet.ether | - | 12 |
| $CH_2NHCOCH_2$- 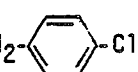 | (α+β)158-160 | pet.ether | - | 10 |
| 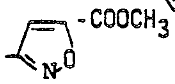 | (α) 96-98 | n-pentane | - | 50 |

Table 2

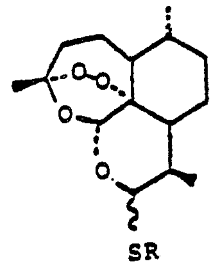

| R | | m.p. (°C) | Solvent for crystallisation | salt | Yield (%) |
|---|---|---|---|---|---|
| $C_2H_5$ | (α) | 51 - 54 | n-pentane | - | 48 |
| $C_2H_5$ | (β) | 95 - 97 | n-pentane | - | 35 |
| $CH(CH_3)_2$ | (α+β) | oil | - | - | 75 |
| $CH_2CH_2OH$ | (α) | oil | - | - | 41 |
| $CH_2COOH$ | (β) | 103-105 | pet.ether (60-80) | - | 47 |
| $CH_2CH_2COOH$ | (β) | oil | - | - | 43 |
| 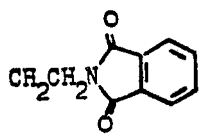 | (α+β) | oil | - | - | 43 |
| $CH_2CH_2NH_2$ | (α+β) | 96 - 98 | methylene chloride/ diisopropyl ether | maleate | 28 |
| 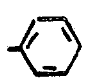 | (α) | 85 - 87 | pet.ether (40-60) | - | 30 |
| 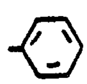 | (β) | 95 - 97 | pet.ether | - | 22 |

**Table 2** (Contd.)

| R | m.p. (°C) | Solvent for crystallisation | salt | Yield (%) |
|---|---|---|---|---|
| 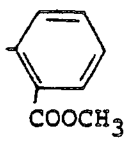 (α) | 130-131 | n-pentane | - | 20 |
| 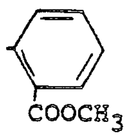 (β) | 135-137 | n-pentane | - | 42 |
| 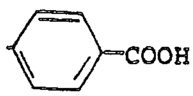 (β) | 63- 65 | methylen chloride/ pet.ether | ½$H_2O$ | 25 |
| 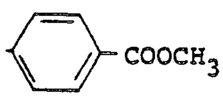 (α) | 145-147 | pet.ether | - | 11 |
| 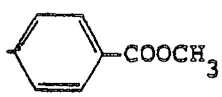 (β) | 152-153 | pet.ether | - | 35 |
| 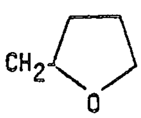 (α) | 114-116 | pet.ether | - | 14 |
| 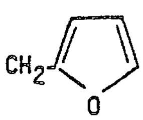 (α) | 90- 92 | n-pentane | - | 48 |
| 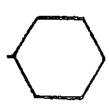 (α) | 98-100 | pet.ether | - | 62 |

Table 2 (Contd.)

| R | m.p. (°C) | Solvent for crystallisation | salt |
|---|---|---|---|
| 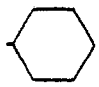 (β) | 107-109 | pet.ether | - |
| 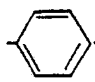-CH=CH-COOH (β) | 118-120 | methylene chloride/ pet.ether | - |
| $CH_2CH=CH$-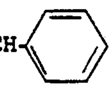 (α) | 145-147 pet.ether | methylen chloride/ | - |

Further preferred compounds of the instant invention are those in which X-R is a residue of the following formulae:

$-OC(CH_3)(C_2H_5)C{\equiv}CH$, $-OCH(CH_3)COCH_3$ and O-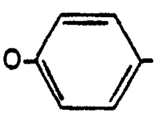-$COCH_3$.

Particularly preferred compounds of the invention are:
3α,12α-epoxy-3,4,5,5aα,6,7,8,8aα,9,10,12β,12a-dodecahydro-10β-ethylthio-3β,6α,9β-trimethylpyrano(4,3-j)-(1,2)benzodioxepin,
3α,12α-epoxy-3,4,5,5aα,6,7,8,8aα,9,10,12β,12a-dodecahydro-10α-ethylthio-3β,6α,9β-trimethylpyrano(4,3-j)-(1,2)benzodioxepin,
3α,12α-epoxy-3,4,5,5aα,6,7,8,8aα,9,10,12β,12a-dodecahydro-10(α + β)-isopropylthio-3β,6α,9β-trimethylpyrano(4,3-j)(1,2)benzodioxepin,
3α,12α-epoxy-3,4,5,5aα,6,7,8,8aα,9,10,12β,12a-dodecahydro-10α-phenylthio-3β,6α,9β-trimethylpyrano(4,3-j)-(1,2)benzodioxepin,
3α,12α-epoxy-3,4,5,5aα,6,7,8,8aα,9,10,12β,12a-dodecahydro-10α-cyclohexylthio-3βm6α,9β-trimethylpyrano-(4,3-j)(1,2)benzodioxepin,
3α,12α-epoxy-3,4,5,5aα,6,7,8,8aα,9,10,12β,12a-dodecahydro-10β-cyclohexylthio-3β.6α,9β-trimethylpyrano-(4,3-j)(1,2)benzodioxepin,
4-[(3α,12α-epoxy-3,4,5,5aα,6,7,8,8aα,9,10,12β,12a-dodecahydro-3β,6α,9β-trimethylpyrano(4,3-j)(1,2)-benzodioxepin-10β-yl)thio]cinamic acid,
3α,12α-epoxy-3,4,5,5aα,6,7,8,8aα,9,10,12β,12a-dodecahydro-10α-[1,3-bis(isopropoxypropyl-2]oxy-3β,6α,9β-trimethylpyrano(4,3-j)(1,2)benzodioxepin,
5-[3α,12α-epoxy-3,4,5,5aα,6,7,8,8aα,9,10,12β,12a-dodecahydro-3β,6α,9β-trimethylpyrano(4,3-j)(1,2)-benzodioxepin-10-oxy]methyl-3-(4-carboxyphenyl)isoxazole,
3α,12α-epoxy-3,4,5,5aα,6,7,8,8aα,9,10,12β,12a-dodecahydro-10β-(2-methyl-3-butynyl-2-oxy)-3β,6α,9β-trimethylpyrano(4,3-j)(1,2)benzodioxepin,
3α,12α-epoxy-3,4,5,5aα,5,7,8,8aα,9,10,12β,12a-dodecahydro-10β-(3-butynyl-2-oxy)-3β,6α,9β-trimethylpyrano(4,2-j)(1,2)benzodioxepin,
5-(3α,12α-epoxy-3,4,5,5aα,6,7,8,8aα,9,10,12β,12a-dodecahydro-3β,6α,9β-trimethylpyrano(4,3-j)(1,2)-benzodioxepin-10β-yl]oxymethyl-3-bromo-isoxazole, and
5-(3α,12α-epoxy-3,4,5,5aα,6,7,8,8aα,9,10,12β,12a-dodecahydro-3β,6α,9β-trimethylpyrano(4,3-j)(1,2)-benzodioxepin-10β-yl]oxymethyl-3-chloro-isoxazole.

The process for the preparation of compounds of the invention comprises treating compounds of the formula II with compounds of the

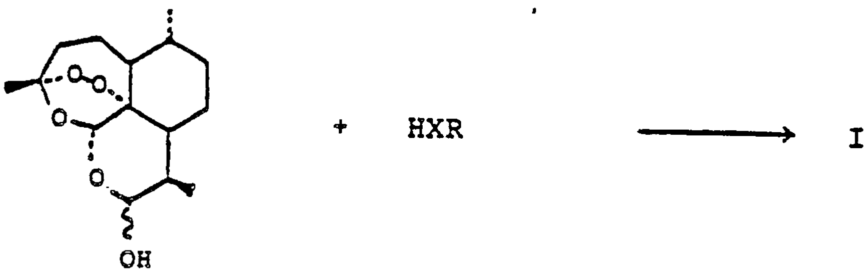

\+ HXR I

II III

formula III wherein X and R have the same meaning as defined above, preferably in the presence of $BF_3$-etherate at a temperature of 0° C - 10° C under stirring for half an hour to six hours. The reaction is preferably carried out in organic solvents such as benzene, chloroform. For completion of the reaction, the reaction mixture may be heated to the boiling point of the solvent used. The compounds of the invention are isolated by diluting the reaction mixture with water, separating the organic layer, washing it with water, concentrating the organic layer and purifying it by flash column chromatography using silica gel column. Compounds of the formula II are obtained by the procedure reported in the literature (Acta. Chim. Sinica 37, 129 (1979)).

The compounds of formula I may be administered in different manners, preferably perorally or parenterally in doses ranging from 2.5 to 100 mg/kg of body weight. As antimalarial drugs dosage unit forms such as dragees or capsules for oral administration or solutions and suspensions respectively for injections, each containing 100 to 400 mg of active substance are preferred. Such dosage units are administered once to three times daily depending upon the condition of the patient.

For oral administration, there may be used in particular tablets, dragees, capsules, powders or granules which contain the active substance together with the usual carriers, adjuvants and/or excipients such as starch, cellulose powder, talcum, magnesium stearate, sugar, gelatin, calcium, carbonate, finely divided silicic acid, carboxymethyl cellulose or similar substances.

For parenteral administration, in particular for intramuscular injections, there may be used sterile suspensions for example oily suspensions prepared with the use of sesame oil, vegetable oil, castor oil or synthetic triglycerides, optionally with simultaneous use of surface active substances such as sorbitan fatty acid esters. Furthermore, there may also be used aqueous suspensions prepared for example with the use of ethoxylated sorbitan fatty acid esters, optionally with addition of thickeners such as polyethylene glycol or carboxymethyl cellulose.

**Biological Evaluation Methodology**

The evaluation of blood-schizontocidal activity "28-day test" described by Raether and Fink [W. H. O. Report on the Scientific Working Group on the Chemotherapy in Malaria, TDR/Chemal 3rd Review, 85.3, Geneva, 3-5 June 1985 and references contained therein] was followed.

**Mice:** All experiments were carried out in random bred male and female Swiss mice obtained from the Hoechst India Limited breeding house at Mulund, Bombay. The animals were free from Eperythrozoon coccoides. The animals received food pellets and water ad lib and were kept at 22-25° C room temperature.

Parasite: Plasmodium berghei K-173 strain drug-sensitive and P. berghei (NS) moderately resistant to chlorquine were obtained from the London School of Hygiene and Tropical Medicines. The strains produce lethal infection at 1 x $10^7$ parasitized red blood cells per mouse when inoculated intraperitoneally.

**Administration of compounds:** The compounds were administered orally or subcutaneously as per methods described by Raether and Fink [W. H. O. Report of the Scientific Working Group on the Chemotherapy in Malaria, TDR/Chemal 3rd Review, 85.3, Geneva, 3-5 June 1985 and references contained therein].

Compounds of the invention were homogenized in double refined Kardi oil or peanut oil or corn oil with one or two drops of polyoxyethylenesorbitan monooleate (Tween-80, Sigma Chanicallo, England) and such suspensions were used for subcutaneous inoclutaion in mice. Drugs were administered for 5 days. 1st dosing was done within 2 hours of infection (D+0) followed by D+1, D+2, D+3 and D+4.

**Observation of the treated mice:** The blood smears were prepared at different intervals from D+4

and continued up to D+28. Blood smears were drawn from the terminal end of the tail and stained in Giemsa. Mice which were free from P. berghei on D+28 were considered as completely cured.

Results obtained with the compounds of Formula I of the invention are listed in Table 3.

**Table 3**

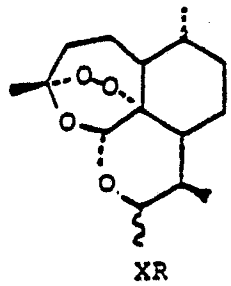

| XR | | Dose mg/kg x 5 | Route of administration s.c. or p.o. | Activity No. of Animals treated/cured D+7 | No. of Animals treated/cured D+28 |
|---|---|---|---|---|---|
| 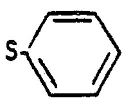 | (α) | 5.0 | s.c. | 11/11 | 11/11 |
| | | 2.5 | s.c. | 6/6 | 6/6 |
| | | 25 | p.o. | 6/6 | 4/6 |
| 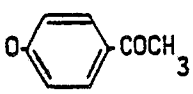 | (β) | 5.0 | s.c. | 6/6 | 4/6 |
| | | 2.5 | s.c. | 1/6 | 0/6 |
| | | 20 | p.o. | 5/5 | 4/8 |
| $OC(CH_3)(C_2H_5)C\equiv CH$ | (β) | 5.0 | s.c. | 6/6 | 4/6 |
| | | 10 | s.c. | 6/6 | 4/6 |
| 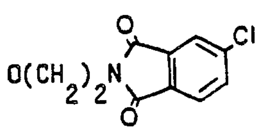 | (β) | 10.0 | s.c. | 6/6 | 4/6 |
| $SC_2H_5$ | (α) | 5.0 | s.c. | 12/12 | 11/12 |
| | | 20.0 | p.o. | 5/5 | 1/5 |
| $SC_2H_5$ | (β) | 5.0 | s.c. | 18/18 | 16/18 |
| | | 2.5 | s.c. | 9/15 | 2/16 |
| | | 50.0 | p.o. | 6/6 | 4/6 |
| $OCH(CH_3)COCH_3$ | (β) | 5.0 | s.c. | 6/6 | 6/6 |

**Table 3 (Contd.)**

| XR | | Dose mg/kg x 5 | Route of administration s.c. or p.o. | Activity No. of Animals treated/cured D+7 | No. of Animals treated/cured D+28 |
|---|---|---|---|---|---|
| $SCH(CH_3)_2$ | (α+β) | 10.0 | s.c. | 6/6 | 1/6 |
| | | 5.0 | s.c. | 3/6 | 0/6 |
| $OCH(CH_3)C{\equiv}CH$ | (β) | 5.0 | s.c. | 6/6 | 6/6 |
| $OC(CH_3)_2C{\equiv}CH$ | (β) | 5.0 | s.c. | 6/6 | 6/6 |
| 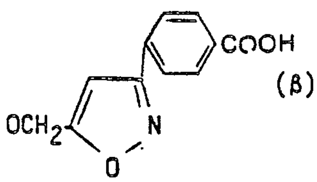 | (β) | 50.0 | s.c. | 8/8 | 8/8 |
| 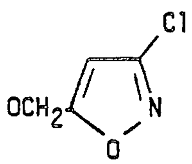 | (β) | 5.0 | s.c. | 5/5 | 5/5 |
| 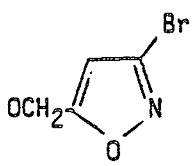 | (β) | 5.0 | s.c. | 6/6 | 5/6 |
| $OCH[CH_2OCH(CH_3)_2]_2$ | (α) | 5.0 | s.c. | 6/6 | 4/6 |
| 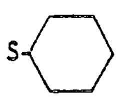 | (α) | 5.0 | s.c. | 6/6 | 6/6 |
| | | 25.0 | p.o. | 6/6 | 6/6 |
| 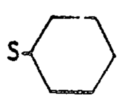 | (β) | 5.0 | s.c. | 6/6 | 6/6 |
| 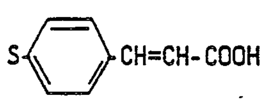 | (β) | 50.0 | s.c. | 6/6 | 6/6 |

The following examples illustrate the invention but do not limit the scope of the invention.

**Example 1**

3α,12α-Epoxy-3,4,5,5aα,6,7,8,8aα,9,10,12β,12a-dodecahydro-10β-ethylthio-3β,6α,9β-trimethylpyrano(4,3-j)-(1,2)benzodioxepin

To a solution of dihyroquinghaosu (0.5 g, 0.001 m) and ethanethiol (0.26 ml, 0.0036 m) in 25 ml chloroform was added $BF_3$ etherate (8 drops) at 0°C and after the addition, mixture was stirred in ice bath for additional 15 minutes. The reaction mixture was then diluted with water and organic layer separated and washed thoroughly with water, dried over anhydrous sodium sulphate and concentrated to obtain the residue, an oil which was purified by Flash Chromatography over silica gel using petroleum ether:ethyl acetate (9.6:0.5) as eluants. Concentration of the first few fractions gave the pure solid which recrystallised from n-pentane to give 3α,12α-epoxy-3,4,5,5aα,6,7,8,8aα,9,10,12β,12a-dodecahydro-10β-ethylthio-3β,6α,9β-trimethylpyrano(4,3-j)(1,2)benzodioxepin, m. p. 95-97°C in 35 % yield.

## Example 2

3α,12α-Epoxy-3,4,5,5aα,6,7,8,8aα,9,10,12β,12a-dodecahydro-10α-ethylthio-3β,6α,9β-trimethylpyrano(4,3-j)-(1,2)benzodioxepin.

Procedure described in Example 1 was followed. Residue obtained after concentrating organic layer was purified using flash column chromatography on silica gel using petroleum ether:ethyl acetate (95.5:0.5) as eluant. First few fractions gave 3α,12α-epoxy-3,4,5,5aα,6,7,8,8aα,9,10,12β,12a-dodecahydro-10β-ethylthio-3β,6α,9β-trimethylpyrano(4,3- j) (1,2)benzodioxepin which was separated out and elution continued with the same eluant to obtain in subsequent fractions 3α,12α-Epoxy-3,4,5,5aα,6,7,8,8aα,9,10,12β,12a-dedocahydro10α-ethylthio-3β,6α,9β-trimethylpyrano-(4,3-j)(1-2)benzo-dioxepin as a solid which recrystallised from n-pentane to give crystals, m.p. 51-54°C in 48 % yield.

## Example 3

3α,12α-Epoxy-3,4,5,5aα,6,7,8,8aα,9,10,12β,12a-dodecahydro-10(α + β)isopropylthio-3β,6α,9β-trimethylpyrano(4,3-j)(1,2)benzodioxepin.

Following the procedure described in Example 1, using isopropylthiol in place of ethanethiol, the compound 3α,12α-epoxy-3,4,5,5aα,6,7,8aα,9,10,12β,12a-dodecahydro-10(α + β)isopropylthio-3β,6α,9β-trimethyl pyrano(4,3-j)(1,2)benzodioxepin was obtained as an oil in 75 % yield.

## Example 4

3α,12α-Epoxy-3,4,5,5aα,6,7,8,8aα,9,10,12β,12a-dodecahydro-10β-phenylthio-3β,6α,9β-trimethylpyrano(4,3-j)-(1,2)benzodioxepin

Following the procedure described in Example 1 using thiophenol in place of ethanethiol, the compound 3α,12α-Epoxy-3,4,5,5aα,6,7,8,8aα,9,10,12β,12a-dodecahydro-10β-phenylthio-3β,6α,9β-trimethylpyrano(4,3-j)-(1,2)benzodioxepin was obtained in 22 % yield, m.p. 95 - 97°C.

Following the procedure described in the above examples, the compounds reported in Tables 1 and 2 were prepared similarly using appropriate nucleophile in place of ethanethiol.

## Claims

1. 10-Substituted ethers and thioether derivates of dihydroquinghaosu of the formula I

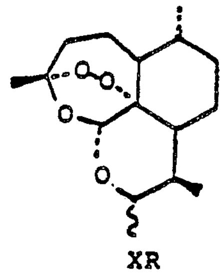

wherein X stands for oxygen, sulphur, SO or $SO_2$ when X stands for sulphur, SO or $SO_2$, R stands for $C_1$-$C_8$ alkyl, $C_4$-$C_8$ cycloalkyl, substituted alkyl, alkenyl, substituted alkenyl, alkynyl, substituted alkynyl,
aryl,
aralkyl,
alkylsulfinyl,
heterocyclic alkyl
a group

$$\underset{R^1}{(CH)}_{m_1}-(CH_2)_{n_1}-\underset{R^2}{(CH)}_{m_2}-(CH_2)_{n_2}-\underset{R^3}{(CH_2)}_{m_3}-(CH_2)_{n_3}-Y$$

wherein $R_1$ stands for hydrogen, alkyl; $R_2$ and $R_3$ stand for hydrogen, hydroxy, alkyl
Y, stands for nitrile, aryl or a group

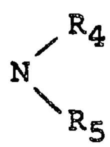

wherein $R_4$, $R_5$ when they are same stand for hydrogen, alkyl, substituted alkyl, when $R_4$ stands for hydrogen, $R_5$ stands for alkyl, substituted alkyl, aryl, aralkyl; when $R_4$ and $R_5$ together with the nitrogen atom to which they are attached form a heterocycle, this heterocycle may contain an additional heteroatom and may optionally be substituted at one or more places, $m_1$-$m_3$ stand for 0 or 1 and $n_1$-$n_3$ stand for integer 0-9 with the proviso that $m_1$-$m_3$ and $n_1$-$n_3$ do not stand simultaneously for 0,
when X stands for oxygen, R stands for 3-hydroxypropyl, acetoxy ethyl,
oxypropyl,
2,3-oxypropyl,
bis isopropoxypropyl,
ethylnitrile,
3-methyl-1-pentynyl,
heterocyclic alkyl,
2-hydroxyethyloxy ethyl
a group

$$\underset{R^1}{(CH)}_{m_1}-(CH_2)_{n_1}-\underset{R^2}{(CH)}_{m_2}-(CH_2)_{n_2}-\underset{R^3}{(CH)}m_3-(CH_2)_{n_3}-Y$$

which has the meaning as defined above or an aryl group and pharmaceutically acceptable salts thereof

with the exception of those compounds in which X is O and R is benzyl, 4-methoxybenzyl, 4-carboxybenzyl, 3-fluorobenzyl, phenyl and phenyl substituted by methyl, methyloxy, ethyloxy, halogen, trichloromethyl and tribromomethyl.

2. Substituted ethers and thioether derivatives of dihydroquinghaousu of the formula I as claimed in claim I wherein X stands for O and R stands for

$CH_2CN$

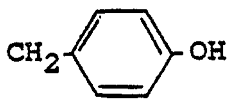

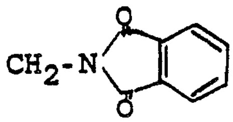

$CH_2COCH_3$,

$CH_2CH_2CN$,

$CH_2CH_2CH_2OH$,

$CH_2CH_2CH_2NH_2$,

$CH_2$-CH-$CH_3$ ,
OH

$CHCH_2CH_2NH_2$ ,
$CH_3$

$CH_2CHCH_2N(C_2H_5)_2$,
OH

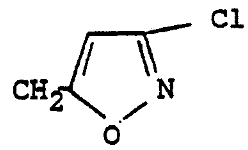

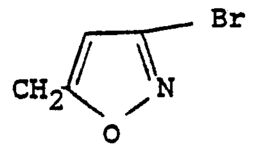

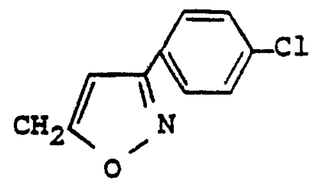

$CH_2CH_2OCH_2CH_2OH$,

$CH_2CH_2NH_2$,

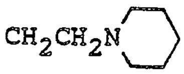

,

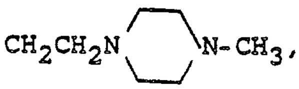

,

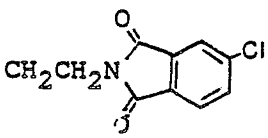

,

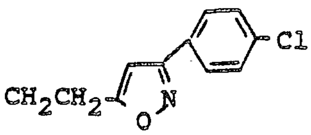

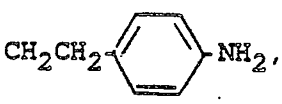

,

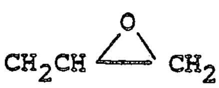

,

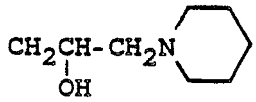

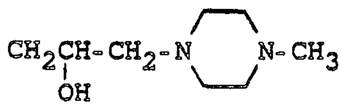

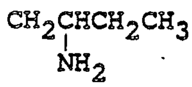

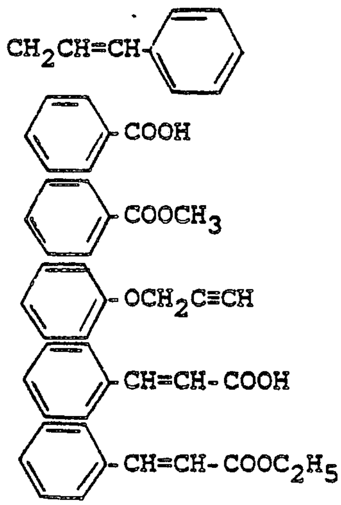

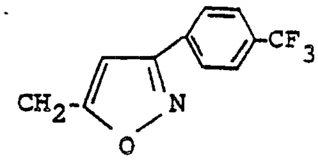

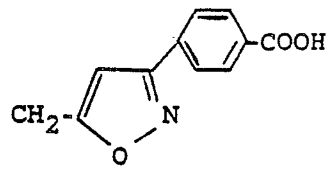

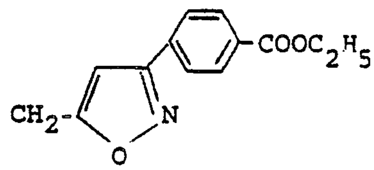

$CH_2C{\equiv}CCH_2OH$

$CH(CH_3)C{\equiv}CH$

$C(CH_3)_2C{\equiv}CH$

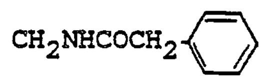

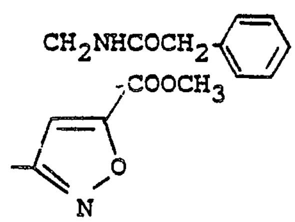

$C(CH_3)(C_2H_5)C{\equiv}CH$

$CH(CH_3)COCH_3$

or

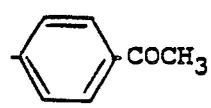

or X stands for S and R stands for

$C_2H_5$

$CH(CH_3)_2$

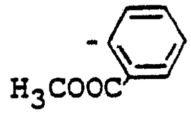

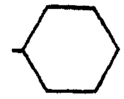

$CH_2CH_2OH$

$CH_2COOH$

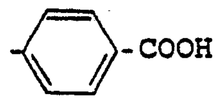

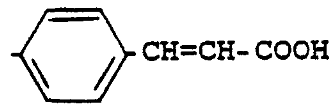

$CH_2CH_2COOH$

$CH_2CH_2NH_2$

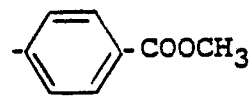

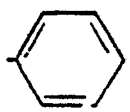

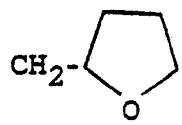

or 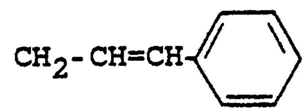

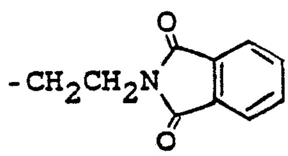

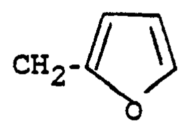

and pharmaceutically acceptable salts thereof.

3. 10-Substituted ethers and thioether dervatives of dihydroquinghaosu which have the following formulas:

3α,12α-epoxy-3,4,5,5aα,6,7,8,8aα,9,10,12β,12a-dodecahydro-10β-ethylthio-3β,6α,9β-trimethylpyrano(4,3-j)-(1,2)benzodioxepin,

3α,12α-epoxy-3,4,5,5aα,6,7,8,8aα,9,10,12β,12a-dodecahydro-10α-ethylthio-3β-6α,9β-trimethylpyrano(4,3-j)(1-2)benzodioxepin,

3α,12α-epoxy-3,4,5,5aα,6,7,8,8aα,9,10,12β,12a-dodecahydro-10α-phenylthio-3β,6α,9β-trimethylpyrano(4,3-j)-(1,2)benzodioxepin,

3α,12α,epoxy-3,4,5,5aα,6,7,8,8aα,9,10,12β,12a-dodecahydro-10(α + β)-isopropylthio-3β,6α,9β-trimethylpyrano(4,3-j)(1,2)benzodioxepin,

3α,12α-epoxy-3,4,5,5aα,6,7,8,8aα,9,10,12β,12a-dodecahydro-10α-cyclohexylthio-3β6α,9β-trimethylpyrano-(4,3-j)(1,2)benzodioxepin,

3α,12α-epoxy-3,4,5,5aα,6,7,8,8aα,9,10,12β,12a-dodecahydro-10β-cyclohexylthio-3β.6α,9β-trimethylpyrano-(4,3-j)(1,2)benzodioxepin,

4-[(3α,12α-epoxy-3,4,5,5aα,6,7,8,8aα,9,10,12β,12a-dodecahydro-3β,6α,9β-trimethylpyrano(4,3-j)(1,2)-benzodioxepin-10β-yl)thio]cinnamic acid,

3α,12α-epoxy-3,4,5,5aα,6,7,8,8aα,9,10,12β,12a-dodecahydro-10α-[1,3-bis(isopropoxypropyl-2]oxy-3β,6α,9β-trimethylpyrano(4,3-j)(1,2)benzodioxepin,

5-[3α,12α-epoxy-3,4,5,5aα,6,7,8,8aα,9,10,12β,12a-dodecahydro-3β,6α,9β-trimethylpyrano(4,3-j)(1,2)-benzodioxepin-10-oxy]methyl-3-(4-carboxyphenyl)isoxazole,

3α,12α-epoxy-3,4,5,5aα,6,7,8,8aα,9,10,12β,12a-dodecahydro-10β-(2-methyl-3-butynyl-2-oxy)-3β,6α,9β-trimethylpyrano(4,3-j)(1,2)benzodioxepin,

3α,12α-epoxy-3,4,5,5aα,5,7,8,8aα,9,10,12β,12a-dodecahydro-10β-(3-butynyl-2-oxy)-3β,6α,9β-trimethylpyrano(4,2-j)(1,2)benzodioxepin,

5-(3α,12α-epoxy-3,4,5,5aα,6,7,8,8aα,9,10,12β,12a-dodecahydro-3β,6α,9β-trimethylpyrano(4,3-j)(1,2)-

benzodioxepin-10β-yl]oxymethyl-3-bromo-isoxazole, or
5-(3α,12α-epoxy-3,4,5,5aα,6,7,8,8aα,9,10,12β,12a-dodecahydro-3β,6α,9β-trimethylpyrano(4,3-j)(1,2)-benzodioxepin-10β-yl]oxymethyl-3-chloro-isoxazole and pharmaceutically acceptable salts thereof.

4. A process for the production of 10-substituted ethers and thioether derivatives of dihydroquinghaosu of the formula I as claimed in claims 1-3, wherein a compound of the formula II

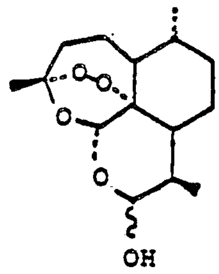

II

is reacted with a compound of the formula HXR, wherein X and R have the meanings given in claims 1 or 2.

5. A pharmaceutical composition, which contains at least one compound as claimed in one or more of claims 1-3, optionally together with pharmaceutically acceptable carriers, adjuvants and/or excipients.

6. A pharmaceutical composition as claimed in claim 5 which contains an amount of at least compound as claimed in one or more of claims 1-3 in an amount which is active against protozoa.

7. A pharmaceutical as claimed in claims 5 or 6 for oral administration.

8. A process for the production of a pharmaceutical composition as claimed in claims 5 to 7, wherein the active compound is, optionally together with pharmaceutically acceptable carriers, adjuvants and/or excipients, converted into a form suitable for administration.

9. The use of a compound as claimed in one or more of claims 1-3 for the treatment and/or prophylaxis of diseases which are caused by infections with protozoa.

10. The use of a compound as claimed in one or more of claims 1 - 3 for the treatment and/or prophylaxis of Malaria.

11. The use of a compound as claimed in one or more of claims 1 - 3 for the treatment and/or prophylaxis of diseases which are caused by infections with Entamoeba histolytica or Eimera tenella.

12. A method for treating diseases, which are caused by infections with protozoa, wherein an effective amount of a compound as claimed in one or more of claims 1-3 is administered.

Claims for the following Contracting States: ES

1. A process for the production of 10-substituted ethers and thioether derivatives of dihydroquinghaosu of the formula I

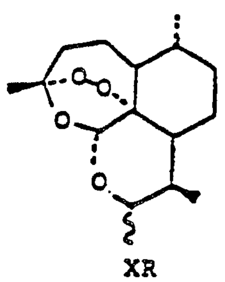

I

wherein X stands for oxygen, sulphur, SO or $SO_2$ when X stands for sulphur, SO or $SO_2$, R stands for $C_1$-$C_8$ alkyl, $C_4$-$C_8$ cycloalkyl, substituted alkyl, alkenyl, substituted alkenyl, alkynyl, substituted alkynyl,
aryl,
aralkyl,
alkylsulfinyl,
heterocyclic alkyl
a group

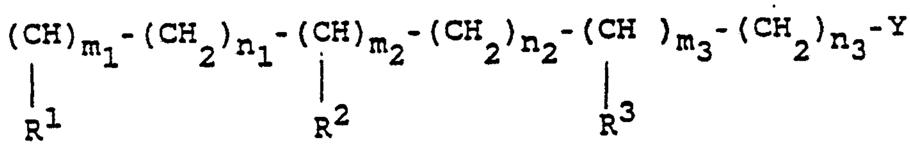

wherein $R_1$ stands for hydrogen, alkyl; $R_2$ and $R_3$ stand for hydrogen, hydroxy, alkyl
Y, stands for nitrile, aryl or a group

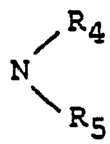

wherein $R_4$, $R_5$ when they are same stand for hydrogen, alkyl, substituted alkyl, when $R_4$ stands for hydrogen, $R_5$ stands for alkyl, substituted alkyl, aryl, aralkyl; when $R_4$ and $R_5$ together with the nitrogen atom to which they are attached form a heterocycle, this heterocycle may contain an additional heteroatom and may optionally be substituted at one or more places, $m_1$-$m_3$ stand for 0 or 1 and $n_1$-$n_3$ stand for integer 0-9 with the proviso that $m_1$-$m_3$ and $n_1$-$n_3$ do not stand simultaneously for 0,
when X stands for oxygen, R stands for 3-hydroxypropyl, acetoxy ethyl,
oxypropyl,
2,3-oxypropyl,
bis isopropoxypropyl,
ethylnitrile,
3-methyl-1-pentynyl,
heterocyclic alkyl,
2-hydroxyethyloxy ethyl
a group

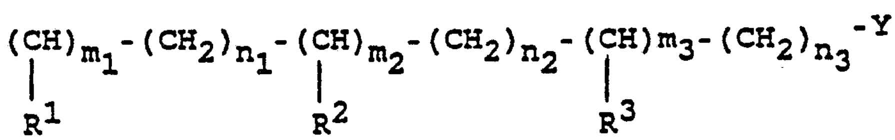

which has the meaning as defined above or an aryl group and pharmaceutically acceptable salts thereof with the exception of those compounds in which X is O and R is benzyl, 4-methoxybenzyl, 4-carboxybenzyl, 3-fluorobenzyl, phenyl and phenyl substituted by methyl, methyloxy, ethyloxy, halogen, trichloromethyl and tribromomethyl.
wherein a compound of the formula II

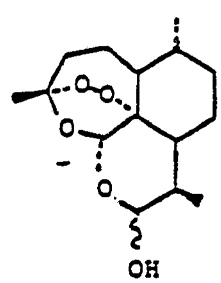

II

is reacted with a compound of the formula HXR, wherein X and R have the meanings as indicated above.

2. A process for the production of substituted ethers and thioether derivatives of dihydroquinghaousu of the formula I as claimed in claim I wherein X stands for O and R stands for

$CH_2CN$

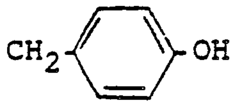

$CH_2CH_2CH_2OH$,

$CH_2CH_2CH_2NH_2$,

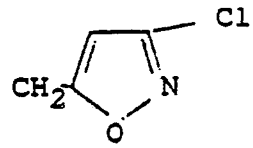

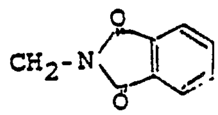

$CH_2COCH_3$,

$CH_2CH_2CN$,

$CH_2CH_2OCH_2CH_2OH$,

$CH_2CH_2NH_2$,

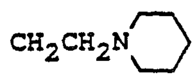

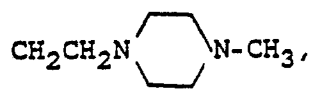

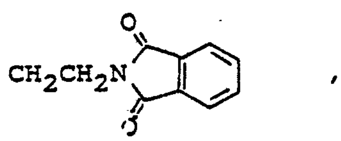

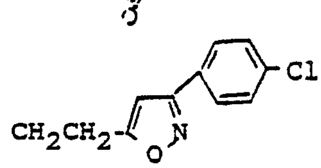

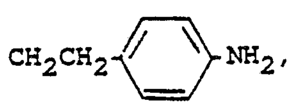

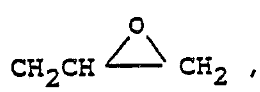

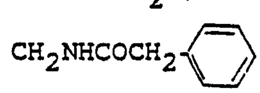

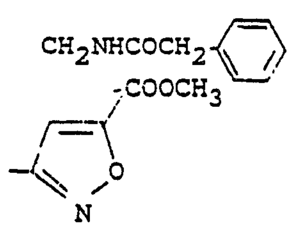

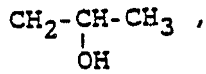

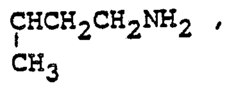

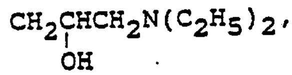

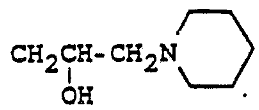

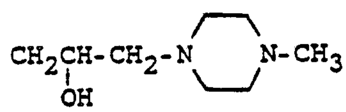

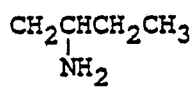

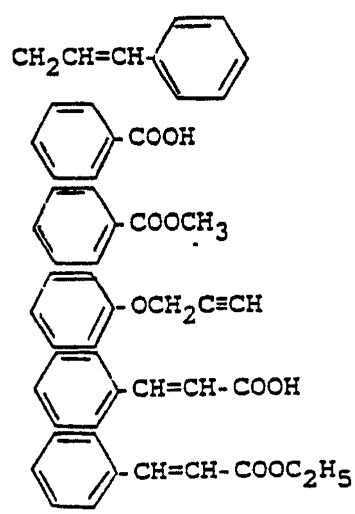

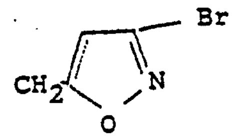

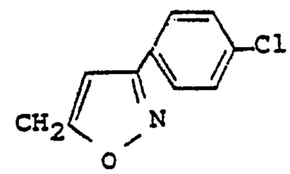

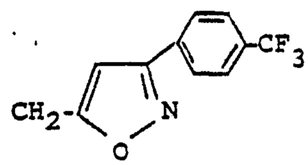

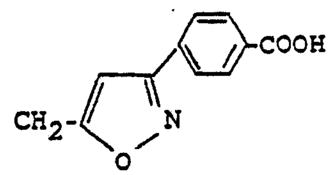

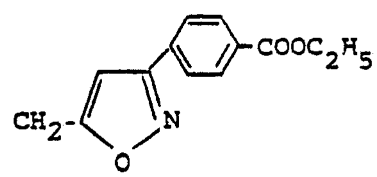

$CH_2C{\equiv}CCH_2OH$

$CH(CH_3)C{\equiv}CH$

$C(CH_3)_2C{\equiv}CH$

$C(CH_3)(C_2H_5)C{\equiv}CH$

$CH(CH_3)COCH_3$

or

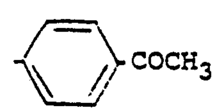

or X stands for S and R stands for

$C_2H_5$

$CH(CH_3)_2$

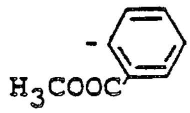

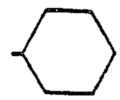

$CH_2CH_2OH$

$CH_2COOH$

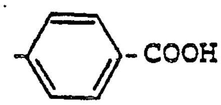

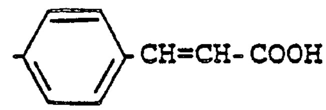

$CH_2CH_2COOH$

$CH_2CH_2NH_2$

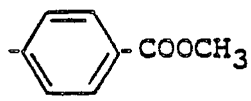

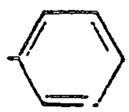

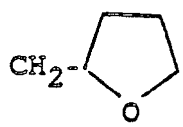

or 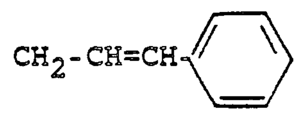

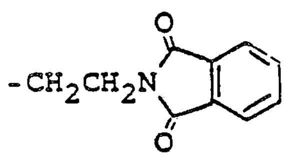

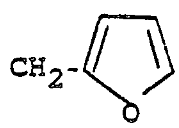

and pharmaceutically acceptable salts thereof.

3. A process as claimed in claim 1 wherein the 10-substituted ethers and thioether dervatives of dihydroquinghaosu which have the following formulas:

3α,12α-epoxy-3,4,5,5aα,6,7,8,8aα,9,10,12β,12a-dodecahydro-10β-ethylthio-3β,6α,9β-trimethylpyrano(4,3-j)-(1,2)benzodioxepin,

3α,12α-epoxy-3,4,5,5aα,6,7,8,8aα,9,10,12β,12a-dodecahydro-10α-ethylthio-3β-6α,9β-trimethylpyrano(4,3-j)(1-2)benzodioxepin,

3α,12α-epoxy-3,4,5,5aα,6,7,8,8aα,9,10,12β,12a-dodecahydro-10α-phenylthio-3β,6α,9β-trimethylpyrano(4,3-j)-(1,2)benzodioxepin,

3α,12α,epoxy-3,4,5,5aα,6,7,8,8aα,9,10,12β,12a-dodecahydro-10(α + β)-isopropylthio-3β,6α,9β-trimethylpyrano(4,3-j)(1,2)benzodioxepin,

3α,12α-epoxy-3,4,5,5aα,6,7,8,8aα,9,10,12β,12a-dodecahydro-10α-cyclohexylthio-3βm6α,9β-trimethylpyrano-(4,3-j)(1,2)benzodioxepin,

3α,12α-epoxy-3,4,5,5aα,6,7,8,8aα,9,10,12β,12a-dodecahydro-10β-cyclohexylthio-3β.6α,9β-trimethylpyrano-(4,3-j)(1,2)benzodioxepin,

4-[(3α,12α-epoxy-3,4,5,5aα,6,7,8,8aα,9,10,12β,12a-dodecahydro-3β,6α,9β-trimethylpyrano(4,3-j)(1,2)-benzodioxepin-10β-yl)thio]cinamic acid,

3α,12α-epoxy-3,4,5,5aα,6,7,8,8aα,9,10,12β,12a-dodecahydro-10α-[1,3-bis(isopropoxypropyl-2]oxy-3β,6α,9β-trimethylpyrano(4,3-j)(1,2)benzodioxepin,

5-[3α,12α-epoxy-3,4,5,5aα,6,7,8,8aα,9,10,12β,12a-dodecahydro-3β,6α,9β-trimethylpyrano(4,3-j)(1,2)-benzodioxepin-10-oxy]methyl-3-(4-carboxyphenyl)isoxazole,

3α,12α-epoxy-3,4,5,5aα,6,7,8,8aα,9,10,12β,12a-dodecahydro-10β-(2-methyl-3-butynyl-2-oxy)-3β,6α,9β-trimethylpyrano-(4,3-j)(1,2)benzodioxepin,

3α,12α-epoxy-3,4,5,5aα,5,7,8,8aα,9,10,12β,12a-dodecahydro-10β-(3-butynyl-2-oxy)-3β,6α,9β-trimethylpyrano(4,2-j )(1,2)benzodioxepin,

5-(3α,12α-epoxy-3,4,5,5aα,6,7,8,8aα,9,10,12β,12a-dodecahydro-3β,6α,9β-trimethylpyrano(4,3-j)(1,2)-benzodioxepin-10β-yl]oxymethyl-3-bromo-isoxazole, or 5-(3α,12α-epoxy-3,4,5,5aα,6,7,8,8aα,9,10,12β,12a-dodecahydro-3β,6α,9β-trimethylpyrano(4,3-j)(1,2)-benzodioxepin-10β-yl]oxymethyl-3-chloro-isoxazole and pharmaceutically acceptable salts thereof.

4. A pharmaceutical composition, which contains at least one compound as claimed in one or more of claims 1-3, optionally together with pharmaceutically acceptable carriers, adjuvants and/or excipients.

5. A process for the production of a pharmaceutical composition as claimed in claim 4, wherein the active compound is, optionally together with pharmaceutically acceptable carriers, adjuvants and/or excipients, converted into a form suitable for administration.

6. The use of a compound as claimed in one or more of claims 1-3 for the treatment and/or prophylaxis of diseases which are caused by infections with protozoa.

7 . The use of a compound as claimed in one or more of claims 1 - 3 for the treatment and/or prophylaxis of Malaria.

8 . The use of a compound as claimed in one or more of claims 1 - 3 for the treatment and/or prophylaxis of diseases which are caused by infections with Entamoeba histolytica or Eimera tenella.

9 . A method for treating diseases, which are caused by infections with protozoa, wherein an effective amount of a compound as claimed in one or more of claims 1-3 is administered.

European Patent Office

# PARTIAL EUROPEAN SEARCH REPORT

which under Rule 45 of the European Patent Convention shall be considered, for the purposes of subsequent proceedings, as the European search report

Application number

EP 89 11 8142

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | MEDICINAL RESEARCH REVIEWS, vol. 7, no. 1, 1987, page 29-52; John Wiley & Sons, Inc. XUAN-DE LUO et al.: "The chemistry, pharmacology, and clinical applications of qinghaosu (Artemisinin) and its derivatives"<br>* Pages 43,44 *<br>-- | 1,5-11 | C 07 D 493/20<br>A 61 K 31/35//<br>(C 07 D 493/20,<br>C 07 D 323:00,<br>C 07 D 321:00,<br>C 07 D 311:00) |
| X | J. MED. CHEM., vol. 30, 1987, pages 2147-2150; Am. Chem. Soc., US; AI JENG LIN et al.: "Antimalarial activity of new water-soluble dihydroartemisinin derivatives"<br>* Page 2147, abstract, scheme I, 5d,7d,8d; page 2148, table 1 * | 1,5-11 | |
| | ./. | | TECHNICAL FIELDS SEARCHED (Int. Cl.4)<br>C 07 D 493/00<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-11

Claims searched incompletely:

Claims not searched: 12

Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 22-12-1989 | BRENNAN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application number

EP 89 11 8142

-2

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X,D | J. MED. CHEM., vol. 31, 1988, pages 645-60; Am. Chem. Soc., US; A. BROSSI et al.: "Arteether, a new antimalarial drug: synthesis and antimalarial properties"<br>* Page 646, figure 1; page 647, table II, table IV *<br>-- | 1,5-11 | |
| X | CHEMICAL ABSTRACTS, vol. 95, no. 17, October 26, 1981, page 696, abstract 150917e; Columbus, Ohio, US; P.L. YU et al.: "Synthesis of artemisinin analogs containing halogen, nitrogen or sulfur atoms" & YAO HSUEH TUNG PAO, 1980, 15(8), 44<br>* Abstract *<br>-- | 1,5-11 | TECHNICAL FIELDS SEARCHED (Int Cl.4) |
| P,X | US-A-4 791 135 (A.J. LIN et al.)<br>* Claims 1,6,7,8 *<br>---- | 1,5-11 | |